(19) Europäisches Patentamt
European Patent Office
Office européen des brevets

(11) **EP 2 836 542 B1**

(12) **EUROPEAN PATENT SPECIFICATION**

(45) Date of publication and mention
of the grant of the patent:
**02.11.2016  Bulletin 2016/44**

(51) Int Cl.:
***C08J 9/04*** (2006.01)   ***C08L 23/04*** (2006.01)
***C08L 33/04*** (2006.01)   ***B01J 20/285*** (2006.01)

(21) Application number: **13776136.7**

(22) Date of filing: **11.03.2013**

(86) International application number:
**PCT/IB2013/051919**

(87) International publication number:
**WO 2013/153468 (17.10.2013 Gazette 2013/42)**

(54) **OPEN-CELLED FOAM WITH SUPERABSORBENT MATERIAL AND PROCESS FOR MAKING THE SAME**

OFFENZELLIGER SCHAUMSTOFF AUS MIT SUPERABSORBIERENDEM MATERIAL UND VERFAHREN ZUR HERSTELLUNG DAVON

MOUSSE À ALVÉOLES OUVERTS COMPRENANT UN MATÉRIAU SUPERABSORBANT ET SON PROCÉDÉ DE FABRICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **12.04.2012   US 201213445754**

(43) Date of publication of application:
**18.02.2015   Bulletin 2015/08**

(73) Proprietor: **Kimberly-Clark Worldwide, Inc.
Neenah, Wisconsin 54956 (US)**

(72) Inventors:
 • **QIN, Jian
  Appleton, Wisconsin 54915 (US)**
 • **CALEWARTS, Deborah
  Winneconne, Wisconsin 54986 (US)**
 • **COLMAN, Charles, W.
  Marietta, Georgia 30062 (US)**
 • **DAY, Jenny, L.
  Woodstock, Georgia 30189 (US)**
 • **UTTECHT, Cathleen, M.
  Menasha, Wisconsin 54952 (US)**

(74) Representative: **Beacham, Annabel Rose
  Dehns
  St Bride's House
  10 Salisbury Square
  London EC4Y 8JD (GB)**

(56) References cited:
  EP-A1- 1 493 411       EP-A2- 0 427 219
  WO-A1-99/58091        WO-A1-2007/075279
  JP-A- H11 309 799     US-A1- 2006 246 272
  US-A1- 2008 275 151    US-B1- 6 673 981

**Description**

FIELD OF THE INVENTION

The present invention relates to an absorbent foam material with an open-celled internal structure capable of delivering both absorbent capacity and fluid intake without the compromise of the foam's characteristic softness and high cushion.

BACKGROUND OF THE INVENTION

Open celled foam is a desirable material for absorbent products such as diapers/training pants, feminine pads, adult incontinence products and the like. Several approaches have been made to incorporate this material into an absorbent product. For example, one approach was to sandwich commercial particulate superabsorbent material between two layers of an open celled foam material. This approach worked when the sandwiched material was in a dry state before absorbing any body fluid. The superabsorbent particles become difficult to be contained, however, after it is wet from body fluid because of the tendency for the superabsorbent particles to swell. The swollen superabsorbent particles eventually pushed out of the foam layers which, led to delamination and resulted in low absorbent core integrity. Another approach was to make absorbent foam using a superabsorbent precursor material. In this approach, a non-cross-linked linear absorbent polymer, which is capable of being cross-linked by certain curing conditions (i.e., heating or high energy ray treatment) after it is shaped to a foam material, was used to replace traditional foam substrate materials such as polyolefin, polyurethane, latex, and other polymers. One drawback to this approach is that the resulting absorbent foam material that is produced exhibits stiffness when the foam is dry due to the glassy nature of the absorbent polymer. Additionally, it produces low absorbent core integrity when the foam is wet due to a high degree of swelling. Another approach was to incorporate particulate superabsorbent material into a close celled foam material, such as thermoplastic foams, produced by a blowing agent. This approach, however, could not utilize the superabsorbent particles due to the nature of the close celled structure which causes inaccessibility of body fluid. Therefore, there is a need to develop absorbent foam material with open celled internal structure to deliver both absorbent capacity and fluid intake capability without compromising the foam material's other properties, such as good superabsorbent containment, absorbent core integrity (wet and dry) and dry softness/high cushion.

EP427219 discloses absorbent latex based foams comprising evenly distributed superabsorbent polymer material.

SUMMARY OF THE INVENTION

The present invention relates to an open-celled foam comprising a particulate superabsorbent polymer material within the voids and pores of said open-celled foam, wherein the open-celled foam is obtainable by frothing (a) an aqueous-based polymer dispersion, said polymer dispersion comprising a polyethylene copolymer and an ethylene-acrylic acid copolymer; and (b) from about 5% to about 15% of a foaming composition, said foaming composition comprising at least a foaming agent and a stabilizing agent to form a frothed foam; adding the superabsorbent polymer material to the frothed foam and then shaping and drying the foam.

The present invention also relates to a process of making the above open-celled foam , said process comprising frothing (a) an aqueous-based polymer dispersion, said polymer dispersion comprising a polyethylene copolymer and an ethylene-acrylic acid copolymer; and (b) from about 5% to about 15% of a foaming composition, said foaming composition comprising at least a foaming agent and a stabilizing agent to form a frothed foam; adding the superabsorbent polymer material to the frothed foam and then shaping and drying the foam.

BRIEF DESCRIPTION OF THE DRAWINGS

FIG. 1 shows a surface and cross-section view of foam without SAM & fiber.
FIG. 2 shows a surface and cross-section view of foam with SAM & fiber.

DETAILED DESCRIPTION OF THE INVENTION

While the specification concludes with the claims particularly pointing out and distinctly claiming the invention, it is believed that the present invention will be better understood from the following description.

All percentages, parts and ratios are based upon the total weight of the compositions of the present invention, unless otherwise specified. All such weights as they pertain to listed ingredients are based on the active level and, therefore; do not include solvents or byproducts that may be included in commercially available materials, unless otherwise specified. The term "weight percent" may be denoted as "wt. %" herein. Except where specific examples of actual measured values are presented, numerical values referred to herein should be considered to be qualified by the word "about".

As used herein, "comprising" means that other steps and other ingredients which do not affect the end result can be added. This term encompasses the terms "consisting of" and "consisting essentially of". The compositions and methods/processes of the present invention can comprise, consist of, and consist essentially of the essential elements and limitations of the invention described herein, as well as any of the additional or optional ingredients, components, steps, or limitations described herein.

"Surfactant" refers to compounds that lower the surface tension of a liquid, the interfacial tension between two liquids, or that between a liquid and a solid. Surfactants may act as detergents, wetting agents, emulsifiers, foaming agents, and dispersants.

As used herein, a "dispersion" refers to a system in which particles are dispersed in a continuous phase of a different composition (or state). A dispersion is classified in a number of different ways including, but not limited to, how large the particles are in relation to the particles of the continuous phase, whether or not precipitation occurs, and the presence of Brownian motion.

As used herein "blow ratio" is the volumetric ratio of gas and liquid in the foam.

"Foam" refers to a substance that is formed by trapping pockets of gas in a liquid or solid. Usually, the volume of gas in the foam is large with thin films of liquid or solid separating the regions of gas.

"Closed Cell Foam" refers to the gas forming discrete pockets within the foam, each completely surrounded by the solid material.

"Open Cell Foam" refers to gas pockets connecting with each other. A bath sponge is an example of an open-cell foam: water can easily flow through the entire structure, displacing the air.

As used herein, "Superabsorbent Material" or "SAM" refers to a water-swellable, water-insoluble organic or inorganic polymer and/or material capable, under the most favorable conditions, of absorbing at least about 10 times its weight and, more suitably, at least about 30 times its weight in an aqueous solution containing from about 0.9 weight percent sodium chloride solution in water.

Preparation of Open-Celled Foam Structure

In order to produce the open celled foam structure of the present invention, an aqueous polymer dispersion is converted into a liquid frothed foam. The polymer dispersion is frothed by a mechanical agitation in the presence of a foaming composition and air. A foaming composition comprises at least a surfactant or a combination of several surfactants that may be added into the dispersion in order to achieve at least four functional goals: foaming capability (to improve dispersion's capability of entrapping total amount of air), stabilizing functionality (to improve containment of the entrapped air during drying step), wetting characteristics (to enhance fluid wettability of the dried foam) and gelation capability in liquid frothed foam (to improve foam resiliency after deformation). A variety of foams can be made depending on the type of foam and functionality sought. For example, a soft and bulky foam may be made by agitating the polymer dispersion with air and a foaming composition comprising at least one surfactant to deliver foaming and stabilization. Alternatively, a soft, bulky and wettable foam may be made by agitating the polymer dispersion with air and a foaming composition comprising at least one surfactant or a combination of several surfactants in order to achieve a frothed foam that is capable of delivering foaming, stabilization and wettability functions.

The surfactants suitable for foaming compositions can be divided into four groups depending on functions: (1) Air Entrapment Agent - used to enhance a liquid's (dispersion, solution, etc.) capability to entrap air which can be measured by determining a "blow ratio." An exemplary list of foaming agents include, but is not limited to, potassium laurate, sodium lauryl sulfate, ammonium lauryl sulfate, ammonium stearate, potassium oleate, disodium octadecyl sulfosuccinimate, hydroxypropyl cellulose, the like, and combinations thereof.; (2) Stabilization Agent - used to enhance the stability of froth's air bubbles against time and temperature. Examples include, but are not limited to, sodium lauryl sulfate, ammonium stearate, hydroxypropyl cellulose, micro- or nano-particles and fibers, the like, and combinations thereof; (3) Wetting Agent - used to enhance the wettability of a dried foam. Examples include, but are not limited to, sodium lauryl sulfate, potassium laurate, disodium octadecyl sulfosuccinimate, the like, and combinations thereof.; (4) Gelling Agent - used to stabilize air bubbles in the froth by causing the dispersion polymer to take the form of a gel which serves to reinforce cell walls. Examples include, but are not limited to, hydroxypropyl cellulose, hydroxyethyl cellulose, carboxymethyl cellulose and other modified cellulose ethers, the like, and combinations thereof. Some surfactants can deliver more than one of the functions listed above. Therefore, although it is possible, it is not necessary to use all four surfactants in a foaming composition at one time. Surfactants can be added in an amount suitable for foaming the composition. For the present invention, surfactants may be added from about 2% to about 20%, by weight of the composition. For any given formulation, there is an inherent capability of entrapping a fixed amount of air. During the frothing process, it is important that there is an adequate amount of air capable of foaming the dispersion. If the air provided is less than what is adequate, the foaming capability of the dispersion formulation may not be fully utilized and thus the foam structure is not considered optimized. On the other hand, if the air supply is higher than what the formulation is capable of handling, larger than normal sized air bubbles will be trapped inside the liquid frothed foam which will change the foam's average pore size and distribution, lead to bursting during the foam's drying step, and ultimately either disrupt the foam's uniformity and/or lead to the creation of defects inside its open-celled structure.

Once the frothed foam has been produced, it is then dried by exposure to an energy source, such as an oven with forced air or an IR heater. Once dried, the resulting foam material will have an open-celled structure wherein the internal pores or voids are interconnected and penetrable. The open-celled foam can be characterized by several structural parameters, for example, density, average pore size and distribution, cell wall thickness, cell shape and uniformity, etc. To control formation of these structural parameters, several factors can be used which include, but are not limited to, type of polymer dispersion chemistries, type of surfactant chemistries, additional amount of the surfactants, dispersion polymer to water ratio (i.e., solids level), frothing equipment (i.e., kitchen-friendly mixers, bench top or commercial scale foaming units), amount of air introduced while mixing, drying rate, temperature and other drying conditions.

Added into the foam's internal pores/voids is a particulate superabsorbent material (SAM). As stated previously, several attempts have been made to combine foam with SAM. The present invention, however, has discovered key elements that allow the resultant open-celled foam to have uniform pore structure, superabsorbent containment, absorbent core integrity, (wet and dry) and dry softness. Thus, it is not enough to simply drop SAM into a foam without appreciating the several factors discovered with the present invention.

It is worth noting a few ineffective attempts in combining foam and SAM so that the present invention can be better appreciated for uncovering a more advantageous solution.

One attempt was pre-swelling of SAM particles before being added into the foam. This approach was not as effective because the swelling prevents an adequate amount of SAM to be added into the foam structure. Pre-swelling was demonstrated in two ways: 1) pre-swelled SAM was added into wet frothed foam with agitation in an effort to uniformly mix the foam and swollen SAM and 2) pre-swelled SAM was added into a layered structure wherein frothed foam was used to sandwich a thin layer of the swollen SAM. Unfortunately, after the foam was dried, the pre-swelling approach resulted in a significant loss of SAM within the foam. Even with a one-to-one volume ratio of frothed foam to swollen SAM added, the final ratio after drying is about 1:20 which constitutes about 5% of SAM materials left within the foam if the SAM is pre-swollen by water to a capacity of about 20 g/g. If the SAM is pre-swollen by water to a capacity of about 30 g/g, the final ratio after drying is about 1:30 which constitutes about 3.3% of SAM within the foam. Although the SAM can occupy large pores within the foam while wet, once dried, the particles of the swollen SAM shrink back to their original size and causes poor particle retention within the foam structure. Additionally, there is a lack of uniform pore size and wide pore size distribution.

Moreover, the polymer dispersion contains a large portion of water. If dry SAM is directly added into the dispersion before it is frothed, the SAM particles will absorb water from the aqueous dispersion which causes the dispersion a loss of foamability. In some cases, such absorption from the aqueous dispersion will cause the dispersion to lose stability and "crash out" of its dispersed polymer. On the other hand, if SAM is added after the frothed foam is produced, shaped and dried; it can only be added onto the surface of the foam or sandwiched into the foam.

One of the key elements of the present invention is that SAM is added into the foam's internal voids and pores during one specific step of foam production process. Specifically, SAM has to be added or mixed into frothed foam when it is produced but not shaped and dried. When SAM is added during this short period of processing time after the frothed foam is just completed by a mechanical mixing procedure but before it is subjected to be molded into any shape and dried, the high viscosity of the frothed foam, though it contains majority of water and has a direct contact with dry SAM, prevents the frothed water from being absorbed by the SAM. If a blow ratio is about 10 to about 30, then the air volume is about 10 to about 30 times as much as water volume in the frothed foam structure. Due to the presence of a huge amount of air, dry SAM surface contacts mostly air and a limited amount of frothed water. The frothed water acts just like solidified water which prevents the SAM from absorbing the frothed water the way it absorbs liquid water. During this specific step, SAM can be uniformly introduced into the frothed foam structure without the chance that it will absorb a significant amount of water. Thus, the dispersion is more stable and increases the attributes attained by the presently claimed invention: uniform pore structure, superabsorbent containment, absorbent core integrity, (wet and dry) and dry softness.

In addition to finding the specific step of foam production process to which the SAM should be added, the chemistry of an aqueous dispersion is also critical. For example, when an alternative polyolefin dispersion that was specially formulated for foam material was used, the dispersion did not mix well with SAM at any amount (such as low as 10 wt%) in order to form a suitable open-celled foam. The dispersion's polymer would be crashed out of the dispersion and became powdery precipitates. However, with a different dispersion that was not necessarily formulated for foam material use, such as HYPOD® 8510, commercially available from Dow Chemical Company, Midland, MI, USA, SAM could be added into the frothed liquid foam at a high level without the occurrence of the polymer crashing out of the dispersion. The difference between the dispersions is the presence of a copolymer of ethylene-acrylic acid, specifically from about 80 wt% of ethylene and from about 20% of acrylic acid in which about 80% acrylic acid co-monomer is neutralized by potassium hydroxide. Such copolymer is commercially available from Dow Chemical Company, as PRIMACOR®. Also commercially available from Dow Chemical Company is the polymer dispersion that the present invention finds suitable as the polymer that can be foamed and mixed with SAM. This polymer dispersion is HYPOD® 8510 , a 42 wt% aqueous dispersion comprising only two polymers: AFFINITY® (a copolymer of ethylene and octene with a molar ratio of 80 to 20) and PRIMACOR® (a copolymer of ethylene and acrylic acid with a molar ratio of 80 to 20). HYPOD® 8510 comprises 40 wt% PRIMACOR®, 60 wt% AFFINITY®.

It is well known that any dispersion can become instable (causing the polymer to crash out) if its emulsifier(s) cannot function properly. Emulsifiers tend to be sensitive to ionic strength (or hardness) of the liquid phase. Superabsorbent polymers contain a lot of ions such as sodium cations ($Na^+$) and carboxyl anions ($-COO^-$). When the superabsorbent particles are added into a liquid frothed foam, its ions will be solvated, significantly increase total ionic strength or hardness of the liquid phase and further reduce effectiveness of the emulsifier(s) in the dispersion. The present application has discovered that the presence of PRIMACOR® or other similar chemicals enhances the compatibility with sodium polyacrylate. Thus, if such chemical(s) are added into the dispersion to enhance its stability at high ionic concentration, it becomes more compatible to mix with the superabsorbent polymers during the foam production process. It seems clear that one can produce foam with the addition of superabsorbent particles by simply mixing SAM into frothed polymer dispersion chemistries. The present invention, however, has discovered that it is more than adding the SAM, but it is taking into total account ionic strength, particular chemical copolymers along with concentration and ratios.

Thus, there are two key elements to the present invention. First, the SAM has to be added or mixed with a liquid dispersion after it is frothed into a high viscosity foam and prior to it being shaped and dried. Additionally, the dispersion chemistry requires a compromise of the polymeric ionic component (for example, PRIMACOR®, to enhance the dispersion's stability in a high ionic strength medium. The particulate superabsorbent polymer material of the present invention is from about 5% to about 50% based on total weight of the foam.

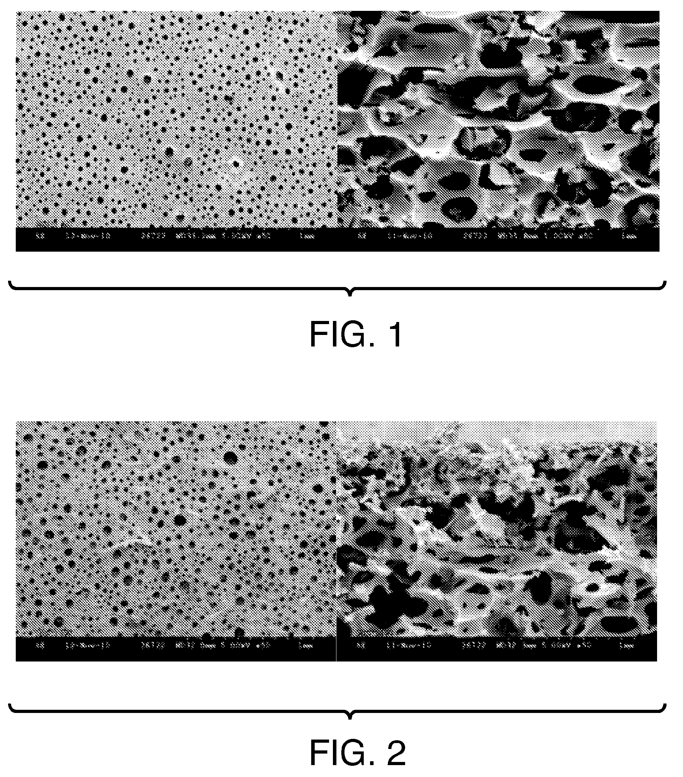

Additionally, the foam may further comprise fibers, non-swellable particles, or combinations thereof, such as, for example FIG.2. Non-swellable particles of the present invention include, but are not limited to, micro-particles, carbon black, silica gels, calcium carbonate, thermally expandable microspheres, additional polymer dispersions, fragrances, anti-bacterials, moisturizers, soothers, medicaments, the like and combinations thereof. Fibers and/or non-swellable particles not only provide enhanced stability to the frothed foam, but they also bring product benefits to the final foam. For example, if carbon black or calcium carbonate powder is added into the foam, the final foam will exhibit improved odor adsorbent properties. Other improved properties due to the addition of the fiber and/or non-swellable particles include, but are not limited to, mechanical strength, elasticity/stretchability, antimicrobial, electrical conductivity, fragrance, thermal isolation, medical effect to skin, etc. The amount of the non-swellable particles and/or fibers that can be used in the foam is from about 5% to about 50% based on total weight of the foam.

It was also discovered that when the dispersion is mixed with an adequate amount of air but still not shaped into its final structure and dried, its overall viscosity is significantly increased. At such a high level of viscosity, even if the superabsorbent particles are introduced, the particles cannot absorb water from the liquid frothed foam. Viscosity plays an important role of significantly slowing down the water absorption rate by the superabsorbent particles so that the liquid frothed foam as well as dispersed superabsorbent particles can be shaped into a uniform final shape of an absorbent foam and dried.

EXAMPLES

The following examples further describe and demonstrate embodiments within the scope of the present invention. The examples are given solely for the purpose of illustration and are not to be construed as limitations of the present invention, as many variations thereof are possible without departing from the spirit and scope of the invention.

Testing Methods

Saturated Capacity Test and the Fluid Intake Rate (FIR) Test as described in US20070135785, published June 14, 2007 to Qin et al, were used to test the absorbent structures accordingly and as shown below.

Saturated Capacity Test

Saturated Capacity is determined using a Saturated Capacity (SAT CAP) tester with a Magnahelic vacuum gage and a latex dam. The overall capacity of each absorbent structure is determined by subtracting the dry weight of each absorbent from the wet weight of that absorbent. The 0.5 psi Saturated Capacity or Saturated Capacity of the absorbent structure is determined by the following formula:

$$\text{Saturated Capacity} = (\text{wet weight} - \text{dry weight}) / \text{dry weight};$$

wherein the Saturated Capacity value has units of grams of fluid/gram of absorbent. For Saturated Capacity, a minimum of three specimens of each sample should be tested and the results averaged. If the absorbent structure has low integrity or disintegrates during the soak or transfer procedures, the absorbent structure can be wrapped in a containment material such as paper toweling, for example SCOTT® paper towels manufactured by Kimberly-Clark Corporation, having a place of business in Neenah, Wisconsin, U.S.A. The absorbent structure can be tested with the overwrap in place and the capacity of the overwrap can be independently determined and subtracted from the wet weight of the total wrapped absorbent structure to obtain the wet absorbent weight.

Fluid Intake Rate Test

The Fluid Intake Rate (FIR) Test determines the amount of time required for an absorbent structure to take in (but not necessarily absorb) a known amount of test solution (0.9 weight percent solution of sodium chloride in distilled water at room temperature). A suitable apparatus for performing the FIR Test is generally described in US20070135785, published June 14, 2007 to Qin et al.

To run the FIR Test, an absorbent sample is weighed and the weight is recorded in grams. Prior to running the FIR test, the aforementioned Saturated Capacity Test is measured on the sample. Thirty percent (30%) of the saturation capacity is then calculated by multiplying the mass of the dry sample (grams) times the measured saturated capacity (gram/gram) times 0.3; e.g., if the test sample has a saturated capacity of 20g of 0.9% NaCl saline test solution/g of test sample and the three inch (7.6 cm) diameter sample weighs one gram, then 6 grams of 0.9% NaCl saline test solution (referred to herein as a first insult) is poured into the top of the cylinder of the apparatus and allowed to flow down into the absorbent sample. (The "Intake Amount" (ml) is the amount of fluid used for each insult). A time period of fifteen minutes is allowed to elapse, after which a second insult equal to the first insult is poured into the top of the cylinder and again the intake time is measured as described above. After fifteen minutes, the procedure is repeated for three more insults. An intake rate (in milliliters/second) for each of the four insults is determined by dividing the amount of solution (e.g., six grams) used for each insult by the intake time measured for the corresponding insult. At least three samples of each absorbent test are subjected to the FIR Test and the results are averaged to determine the intake rate.

Experiment and Results

1. Preparation of Frothed Absorbent Foam

The foam samples were prepared using a KitchenAid® mixer to produce frothed chemistries and coated the frothed chemistries onto spunbond nonwoven prior to a drying process at a temperature near but below foam polymers' melting points. Table 1 below summarizes all the codes prepared:

Table 1 Foam Code List

| Code | Foam Composition | | | | | Additives | | Comment |
| | Dispersion | | Stanfax 320[2] | Stanfax 318[3] | Water | SAM[4] | Cotton[5] | |
| | Type[1] | Amount | | | | | | |
| 2 | HYPOD® 8510 | 250 g | 14.6 g | 11.3 g | 10 g | 20% | | |
| 3 | HYPOD® 8510 | 250 g | 14.6 g | 11.3 g | 10 g | 40% | | |
| 4 | HYPOD® 8510 | 250 g | 14.6 g | 11.3 g | 10 g | 60% | | |
| 7** | A | 235 g | 0g | 0g | 50 g | 60% | | Dispersion crashed out |
| 11** | A | 235 g | 0g | 0g | 50 g | 40% | | Dispersion crashed out |
| 12 | HYPOD® 8510 | 250 g | 14.6 g | 11.3 g | 10 g | 20% | 20% | |

(continued)

| Code | Foam Composition | | | | | Additives | | Comment |
|---|---|---|---|---|---|---|---|---|
| | Dispersion | | Stanfax 320[2] | Stanfax 318[3] | Water | SAM[4] | Cotton[5] | |
| | Type[1] | Amount | | | | | | |
| 13** | PIP | 185 g | 16.7 g | 13.0 g | 70 g | 40% | | Foam structure very weak |
| 14** | PIP | 185 g | 16.7 g | 13.0 g | 70 g | 20% | | Foam structure very weak |
| 15 | VAE | 220 g | 15.3 g | 11.8 g | 40 g | 40% | | |
| 16 | VAE | 220 g | 15.3 g | 11.8 g | 40 g | 20% | 20% | |
| 18* | HYPOD® 8510 | 250 g | 14.6 g | 11.3 g | 10 g | 0% | 0% | |
| 19 | PIP | 32 g | 14.4 g | 11.2 g | 30 g | 20% | 20% | |
| | HYPOD® 8510 | 198 g | | | | | | |

[1] HYPOD® 8510 is a commercial polyolefin dispersion with a solid level of 42 wt% available from Dow Chemical Co.; "A" is a polyolefin dispersion with a solid level of 50 wt% specially designed for foam compositions and is absent ethylene-acrylic acid copolymer; PIP is a polyisoprene dispersion with a solid level of 60 wt% available from Kraton (IR401); VAE is a polyvinyl acetate-ethylene copolymer latex dispersion with a solid level of 50 wt% commercially available from Celanese Emulsions (Dur-o-Set Plus).
[2] Stanfax 320 is an ammonium stearate surfactant commercially available from Para Chem with a solid level of 36 wt%.
[3] Stanfax 318 is a sodium lauryl sulfate surfactant commercially available from Para Chem with a solid level of 27 wt%.
[4] SAM is a commercial available particulate superabsorbent polymer from Evonik Industries (SXM 9500).
[5] Cotton is a cotton linter flocks with an average fiber length of about 0.35 mm produced by International Fiber Corporation. * Code 18 is a control code. ** These codes could not produce uniform foam during the process or the formed foams did not have enough integrity for testing.

Notably, HYPOD® 8510 chemistry is stable for adding superabsorbent particles into its frothed foam structure, while "A" was found to be unsuccessful (see, Codes 2-4 vs. Codes 7 and 11). Kraton IR401 (polyisoprene) dispersion can allow addition of superabsorbent particles during production step but its foam structure is so weak that the foam produced from this chemistry cannot be tested. The only solution to produce a foam with superabsorbent particles with this chemistry is to use a blend of two dispersion, such as PIP and HYPED® 8510 (refer to Code 19). Another type of foam is VAE which is also stable with the addition of superabsorbent particles.

2. Absorbent Properties of the Absorbent Foam

The eight codes of absorbent foam containing either superabsorbent particles and/or cotton linter flocks were tested by several physical and absorbency testing methods, such as Saturation Capacity Test, Fluid Intake Rate Test , wet density and growth measurements which were calculated by the dimensions (length and thickness) of each testing sample after each insult of the FIR Test. Tables 2 to 5 summarize the testing data.

Table 2 Physical Testing Data

| Code | Physical Testing Data | | | Water Drop Intake Time (sec) |
|---|---|---|---|---|
| | Basis Weight (gsm) | Thickness (mm) | Density (g/cc) | |
| 2 | 436 | 5.10 | 0.09 | 5.1 |
| 3 | 520 | 5.13 | 0.10 | 32.6 |
| 4 | 606 | 5.52 | 0.11 | 31.9 |
| 12 | 481 | 5.67 | 0.08 | 3.8 |
| 15 | 471 | 4.38 | 0.11 | 11.7 |

(continued)

| Code | Physical Testing Data | | | Water Drop Intake Time (sec) |
|---|---|---|---|---|
| | Basis Weight (gsm) | Thickness (mm) | Density (g/cc) | |
| 16 | 425 | 4.97 | 0.09 | 43.7 |
| 18* | 379 | 5.17 | 0.07 | 2.8 |
| 19 | 476 | 6.00 | 0.08 | 8.8 |
| Note: * Code 18 is a control code. | | | | |

Conclusions from Table 2:

The addition of superabsorbent particles makes foam surface more hydrophobic as water drop intake time increases.

The addition of cotton linter flock fiber enhances foam's hydrophilicity as water drop intake time decreases.

Table 3 Absorbency Testing Data (I)

| Code | Sat. Cap (g/g) | Fluid Intake Test | | | | |
|---|---|---|---|---|---|---|
| | | Intake Amount (ml) | 1st Intake Rate (ml/s) | 2nd Intake Rate (ml/s) | 3rd Intake Rate (ml/s) | 4th Intake Rate (ml/s) |
| 2 | 4.33 | 3.0 | 0.09 | 0.46 | 0.38 | 0.38 |
| 3 | 8.28 | 6.0 | 0.07 | 0.73 | 0.90 | 0.72 |
| 4 | 10.51 | 8.0 | 0.07 | 0.47 | 0.41 | 0.28 |
| 12 | 5.49 | 4.0 | 0.10 | 1.10 | 0.87 | 0.85 |
| 15 | 6.89 | 4.0 | 0.03 | 0.03 | 0.02 | 0.03 |
| 16 | 2.50 | 2.0 | 0.01 | 0.10 | 0.13 | 0.07 |
| 18* | 0.95 | 0.5 | 0.04 | 0.09 | 0.09 | 0.12 |
| 19 | 3.89 | 3.0 | 0.05 | 1.22 | 1.52 | 1.40 |
| Note: * Code 18 is a control code. | | | | | | |

Conclusions from Table 3

The addition of superabsorbent particles enhances significantly foam's absorbency (i.e., Sat. Cap.) and its enhancement is directly proportional to add-on level of superabsorbent particles. Other than 1st fluid intake, addition of superabsorbent particles enhances foam's fluid intake rate and its enhancement is also directly proportional to add-on level of superabsorbent particles. The addition of cotton linter flock fiber also enhances foam's fluid intake rate (Codes 12 vs. 2). Some polymer chemistry, such as VAE, may not have benefit of improving fluid intake rate after addition of superabsorbent particles into its foam structure (Codes 3 vs. 15 or 12 vs. 16). This may be due to VAE's material elasticity since it is a stretchable polymer. VAE's elastic nature may prevent swollen particles from opening up pore size and capillaries.

Table 4 Absorbency Testing Data (II)

| Code | Sat. Cap (g/g) | Foam Density After Each Insult | | | | |
|---|---|---|---|---|---|---|
| | | Initial Density (g/cc) | 2nd Density (g/cc) | 3rd Density (g/cc) | 4th Density (g/cc) | Final Density (g/cc) |
| 2 | 4.33 | 0.10 | 0.25 | 0.38 | 0.50 | 0.62 |
| 3 | 8.28 | 0.11 | 0.32 | 0.53 | 0.69 | 0.87 |
| 4 | 10.51 | 0.13 | 0.42 | 0.65 | 0.85 | 1.06 |

(continued)

| Code | Sat. Cap (g/g) | Foam Density After Each Insult | | | | |
|---|---|---|---|---|---|---|
| | | Initial Density (g/cc) | 2nd Density (g/cc) | 3rd Density (g/cc) | 4th Density (g/cc) | Final Density (g/cc) |
| 12 | 5.49 | 0.09 | 0.24 | 0.39 | 0.54 | 0.69 |
| 15 | 6.89 | 0.18 | 0.64 | 0.90 | 1.07 | 1.15 |
| 16 | 2.50 | 0.12 | 0.30 | 0.48 | 0.67 | 0.83 |
| 18* | 0.95 | 0.08 | 0.10 | 0.13 | 0.15 | 0.17 |
| 19 | 3.89 | 0.08 | 0.19 | 0.30 | 0.41 | 0.52 |
| Note: * Code 18 is a control code. | | | | | | |

Conclusions from Table 4

The control code has a slight foam density change during multiple fluid insults.

Foams containing superabsorbent particles have significant changes in overall density during fluid insults.

Table 5 Absorbency Testing Data (III)

| Code | Wet Growth (%) | | | | | | | | | | | |
| | Machine Direction | | | | Cross Machine Direction | | | | Z-Direction | | | |
| | @ 30% capacity | @ 60% capacity | @ 90% capacity | @ 120 % capacity | @ 30% capacity | @ 60% capacity | @ 90% capacity | @ 120 % capacity | @ 30% capacity | @ 60% capacity | @ 90% capacity | @ 120 % capacity |
|---|---|---|---|---|---|---|---|---|---|---|---|---|
| 2 | 1.7 | 5.0 | 8.3 | 8.3 | 1.7 | 1.7 | 1.67 | 3.3 | 1.7 | 5.6 | 9.6 | 13.0 |
| 3 | 5.0 | 11.7 | 18.3 | 18.3 | 1.7 | 8.3 | 15.0 | 18.3 | 23.0 | 27.6 | 38.3 | 42.0 |
| 4 | 1.7 | 6.7 | 6.7 | 10.0 | 1.7 | 5.0 | 8.3 | 11.7 | 27.7 | 46.6 | 60.3 | 67.1 |
| 12 | 1.7 | 3.3 | 3.3 | 3.3 | 0.0 | 1.7 | 1.67 | 1.7 | 4.4 | 6.5 | 7.71 | 8.5 |
| 15 | 0.0 | 1.7 | 1.7 | 1.7 | 0.0 | 0.0 | 0.0 | 1.7 | -17.0 | -1.5 | 15.8 | 37.9 |
| 16 | 0.0 | 0.0 | 0.0 | 1.7 | 0.0 | 0.0 | 0.0 | 0.0 | -23.2 | -28.5 | -31.6 | -30.6 |
| 18* | 0.0 | 0.0 | 0.0 | 0.0 | 0.0 | 0.0 | 0.0 | 0.0 | -2.1 | -3.7 | -5.7 | -5.0 |
| 19 | 0.0 | 0.0 | 0.0 | 0.0 | 0.0 | 0.0 | 0.0 | 0.0 | 1.8 | 2.1 | 1.9 | 1.6 |
| Note: * Code 18 is a control code. | | | | | | | | | | | | |

Conclusions from Table 5

The control code has no growth in MD and CD directions and a negative growth in the Z-direction which means part of the foam structure slightly collapses when it is wet.

Foam made of elastomer, such as VAE, also collapses when wet in Z-direction but has minor degree growth in MD and CD directions (Codes 15 and 16).

Foam with low addition level of superabsorbent particles and cotton linter flock fiber exhibits almost no growth in MD and CD direction but slight growth in Z-direction; Foams with high content of superabsorbent particles show growth in all three directions.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm".

**Claims**

1. An open-celled foam comprising a particulate superabsorbent polymer material within the voids and pores of said open-celled foam, wherein the open-celled foam is obtainable by frothing (a) an aqueous-based polymer dispersion, said polymer dispersion comprising a polyethylene copolymer and an ethylene-acrylic acid copolymer; and (b) from about 5% to about 15% of a foaming composition, said foaming composition comprising at least a foaming agent and a stabilizing agent to form a frothed foam; adding the superabsorbent polymer material to the frothed foam and then shaping and drying the foam.

2. The open-celled foam of claim 1 wherein the ethylene-acrylic acid copolymer is from about 80 wt% of ethylene and from about 20% of acrylic acid in which about 80% acrylic acid co-monomer is neutralized by potassium hydroxide.

3. The open-celled foam of claim 1 wherein the foaming agents are selected from the group consisting of potassium laurate, sodium lauryl sulfate, ammonium lauryl sulfate, ammonium stearate, potassium oleate, disodium octadecyl sulfosuccinimate, hydroxypropyl cellulose, and combinations thereof.

4. The open-celled foam of claim 1 wherein the stabilizing agent is selected from the group consisting of sodium lauryl sulfate, ammonium stearate, hydroxypropyl cellulose, micro- or nano-particles, fibers, and combinations thereof.

5. The open-celled foam of claim 1 further comprising a wetting agent selected from the group consisting of sodium lauryl sulfate, potassium laurate, disodium octadecyl sulfosuccinimate, and combinations thereof.

6. The open-celled foam of claim 1 further comprising a gelling agent selected from the group consisting of hydroxypropyl cellulose, hydroxyethyl cellulose, carboxymethyl cellulose and other modified cellulose ethers, and combinations thereof.

7. The open-celled foam of claim 3 wherein the foaming agent is from about 2% to about 10% ammonium stearate.

8. The open-celled foam of claim 4 wherein the stabilizing agent is from about 2% to about 10% sodium lauryl sulfate.

9. The open-celled foam of claim 1 wherein the particulate superabsorbent polymer material is from about 5% to about 50% based on total weight of the foam.

10. The open-celled foam of claim 1 wherein the foam further comprises fibers, non-swellable particles, and combinations thereof.

11. The open-celled foam of claim 10, wherein the non-swellable particles are selected from the group consisting of micro-particles, carbon black, silica gels, calcium carbonate, thermally expandable microspheres, additional polymer dispersions, fragrances, anti-bacterials, moisturizers, soothers, medicaments, and combinations thereof.

12. The open-celled foam of claim 10 wherein the foam comprises from about 5% to about 50% fibers, non-swellable particles, and combinations thereof.

13. A process of making the open-celled foam of claim 1, said process comprising frothing (a) an aqueous-based polymer dispersion, said polymer dispersion comprising a polyethylene copolymer and an ethylene-acrylic acid copolymer; and (b) from about 5% to about 15% of a foaming composition, said foaming composition comprising at least a foaming agent and a stabilizing agent to form a frothed foam; adding the superabsorbent polymer material to the frothed foam and then shaping and drying the foam.

**Patentansprüche**

1. Offenzelliger Schaumstoff, umfassend ein teilchenförmiges superabsorbierendes Polymermaterial innerhalb der Hohlräume und Poren des offenzelligen Schaumstoffs, wobei der offenzellige Schaumstoff durch Schäumen (a) einer Polymerdispersion auf Wasserbasis, wobei die Polymerdispersion ein Polyethylen-Copolymer und ein EthylenAcrylsäure-Copolymer umfasst; und (b) von etwa 5% bis etwa 15% einer schäumenden Zusammensetzung, wobei die schäumende Zusammensetzung mindestens ein Schäumungsmittel und ein Stabilisierungsmittel umfasst, erhältlich ist, um einen geschäumten Schaumstoff zu bilden; Zugeben des superabsorbierenden Polymermaterials zu dem geschäumten Schaumstoff und dann Formgebung und Trocknen des Schaumstoffs.

2. Offenzelliger Schaumstoff nach Anspruch 1, wobei das Ethylen-Acrylsäure-Copolymer aus etwa 80 Gew.-% Ethylen und aus etwa 20 % Acrylsäure ist, in welchem etwa 80 % Acrylsäure-Co-monomer durch Kaliumhydroxid neutralisiert ist.

3. Offenzelliger Schaumstoff nach Anspruch 1, wobei die Schäumungsmittel aus der Gruppe, bestehend aus Kaliumlaurat, Natriumlaurylsulfat, Ammoniumlaurylsulfat, Ammoniumstearat, Kaliumoleat, Dinatriumoctadecylsulfosuccinimat, Hydroxypropylzellulose und Kombinationen davon, ausgewählt sind.

4. Offenzelliger Schaumstoff nach Anspruch 1, wobei das Stabilisierungsmittel aus der Gruppe, bestehend aus Natriumlaurylsulfat, Ammoniumstearat, Hydroxypropylzellulose, Mikro- oder Nanoteilchen, Fasern und Kombinationen davon, ausgewählt ist.

5. Offenzelliger Schaumstoff nach Anspruch 1, weiter umfassend ein Netzmittel, ausgewählt aus der Gruppe, bestehend aus Natriumlaurylsulfat, Kaliumlaurat, Dinatriumoctadecylsulfosuccinimat und Kombinationen davon.

6. Offenzelliger Schaumstoff nach Anspruch 1, weiter umfassend ein Geliermittel, ausgewählt aus der Gruppe, bestehend aus Hydroxypropylzellulose, Hydroxyethylzellulose, Carboxymethylzellulose und anderen modifizierten Zelluloseethern und Kombinationen davon.

7. Offenzelliger Schaumstoff nach Anspruch 3, wobei das Schäumungsmittel von etwa 2 % bis etwa 10 % Ammoniumstearat ist.

8. Offenzelliger Schaumstoff nach Anspruch 4, wobei das Stabilisierungsmittel von etwa 2 % bis etwa 10 % Natriumlaurylsulfat ist.

9. Offenzelliger Schaumstoff nach Anspruch 1, wobei das teilchenförmige superabsorbierende Polymermaterial von etwa 5 % bis etwa 50 %, basierend auf dem Gesamtgewicht des Schaumstoffs, ist.

10. Offenzelliger Schaumstoff nach Anspruch 1, wobei der Schaumstoff weiterhin Fasern, nicht-quellbare Teilchen und Kombinationen davon umfasst.

11. Offenzelliger Schaumstoff nach Anspruch 10, wobei die nicht-quellbaren Teilchen aus der Gruppe, bestehend aus Mikroteilchen, Ruß, Kieselgelen, Calciumcarbonat, thermisch expandierbaren Mikrokugeln, weiteren Polymerdispersionen, Duftstoffen, anti-bakteriellen Mitteln, Feuchthaltemitteln, Linderungsmitteln, Medikamenten und Kombinationen davon, ausgewählt sind.

12. Offenzelliger Schaumstoff nach Anspruch 10, wobei der Schaumstoff von etwa 5% bis etwa 50% Fasern, nicht-quellbare Teilchen und Kombinationen davon umfasst.

13. Verfahren zur Herstellung des offenzelligen Schaumstoffs nach Anspruch 1, wobei das Verfahren umfasst Schäumen (a) einer Polymerdispersion auf Wasserbasis, wobei die Polymerdispersion ein Polyethylen-Copolymer und ein Ethylen-Acrylsäure-Copolymer umfasst; und (b) von etwa 5% bis etwa 15% einer schäumenden Zusammensetzung, wobei die schäumende Zusammensetzung mindestens ein Schäumungsmittel und ein Stabilisierungsmittel umfasst, um einen geschäumten Schaumstoff zu bilden; Zugeben des superabsorbierenden Polymermaterials zu dem geschäumten Schaumstoff und dann Formgebung und Trocknen des Schaumstoffs.

## Revendications

1. Mousse à alvéoles ouvertes comprenant un matériau polymère superabsorbant particulaire à l'intérieur des vides et pores de ladite mousse à alvéoles ouvertes, dans laquelle la mousse à alvéoles ouvertes est susceptible d'être obtenue en faisant mousser (a) une dispersion de polymère à base d'eau, ladite dispersion de polymère comprenant un copolymère de polyéthylène et un copolymère d'éthylène-acide acrylique ; et (b) d'environ 5% à environ 15% d'une composition moussante, ladite composition moussante comprenant au moins un agent moussant et un agent stabilisant pour former une mouse écumée ; en ajoutant le matériau polymère superabsorbant à la mousse écumée et ensuite en formant et séchant la mousse.

2. Mousse à alvéoles ouvertes selon la revendication 1 dans laquelle le copolymère d'éthylène-acide acrylique est d'environ 80% en poids d'éthylène et d'environ 20% d'acide acrylique dans lequel environ 80% de comonomère d'acide acrylique est neutralisé par de l'hydroxyde de potassium.

3. Mousse à alvéoles ouvertes selon la revendication 1 dans laquelle les agents moussants sont sélectionnés dans le groupe constitué du laurate de potassium, du laurylsulfate de sodium, du laurylsulfate d'ammonium, du stéarate d'ammonium, de l'oléate de potassium, de l'octadécyle sulfosuccinimate de disodium, de l'hydroxypropylcellulose, et de combinaisons de ceux-ci.

4. Mousse à alvéoles ouvertes selon la revendication 1 dans laquelle l'agent stabilisant est sélectionné dans le groupe constitué du laurylsulfate de sodium, du stéarate d'ammonium, de l'hydroxypropylcellulose, de micro- ou nano-particules, de fibres, et de combinaisons de ceux-ci.

5. Mousse à alvéoles ouvertes selon la revendication 1 comprenant en outre un agent mouillant sélectionné dans le groupe constitué du laurylsulfate de sodium, du laurate de potassium, de l'octadécyle sulfosuccinimate de disodium, et de combinaisons de ceux-ci.

6. Mousse à alvéoles ouvertes selon la revendication 1 comprenant en outre un agent de gélification sélectionné dans le groupe constitué de l'hydroxypropylcellulose, de l'hydroxyéthylcellulose, du carboxyméthylcellulose et d'autres éthers de cellulose modifiés, et de combinaisons de ceux-ci.

7. Mousse à alvéoles ouvertes selon la revendication 3 dans laquelle l'agent moussant est d'environ 2 % à environ 10% de stéarate d'ammonium.

8. Mousse à alvéoles ouvertes selon la revendication 4 dans laquelle l'agent stabilisant est d'environ 2% à environ 10% de laurylsulfate de sodium.

9. Mousse à alvéoles ouvertes selon la revendication 1 dans laquelle le matériau polymère superabsorbant particulaire est d'environ 5% à environ 50% sur la base du poids total de la mousse.

10. Mousse à alvéoles ouvertes selon la revendication 1 dans laquelle la mousse comprend en outre des fibres, des particules non-gonflables, et des combinaisons de celles-ci.

11. Mousse à alvéoles ouvertes selon la revendication 10 dans laquelle les particules non-gonflables sont sélectionnées dans le groupe constitué de microparticules, de noir de carbone, de gels de silice, de carbonate de calcium, de microsphères thermo-expansibles, de dispersions de polymère supplémentaires, de parfums, d'antibactériens, d'hydratants, de calmants, de médicaments, et de combinaisons de ceux-ci.

12. Mousse à alvéoles ouvertes selon la revendication 10 dans laquelle la mousse comprend d'environ 5% à environ 50% de fibres, de particules non-gonflables, et de combinaisons de celles-ci.

13. Procédé de fabrication de la mousse à alvéoles ouvertes selon la revendication 1, ledit procédé comprenant faire mousser (a) une dispersion de polymère à base d'eau, ladite dispersion de polymère comprenant un copolymère de polyéthylène et un copolymère d'éthylène-acide acrylique ; et (b) d'environ 5% à environ 15% d'une composition moussante, ladite composition moussante comprenant au moins un agent moussant et un agent stabilisant pour former une mousse écumée ; ajouter le matériau polymère superabsorbant à la mousse écumée et ensuite former et sécher la mousse.

FIG. 1

FIG. 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 427219 A

- US 20070135785 A, Qin